# EUROPEAN PATENT SPECIFICATION

(11) **EP 1 136 550 B1**
(45) Date of publication and mention of the grant of the patent: **15.12.2004**
(21) Application number: 99917154.9
(22) Date of filing: 23.04.1999
(51) Int. Cl.: C12N 9/96, C12N 9/10

(54) **METHOD FOR STABILIZING ASPARTATE AMINOTRANSFERASE AND THE CORRESPONDING COMPOSITIONS**
VERFAHREN ZUM STABILISIEREN VON ASPARTATAMINOTRANSFERASE UND DIE ENTSPRECHENDEN ZUSAMMENSETZUNGEN
METHODE DE STABILISATION D'ASPARTATE AMINOTRANSFERASE ET COMPOSITIONS CORRESPONDANTES

(30) Priority: 24.04.1998 JP 13115998
(43) Date of publication of application: 26.09.2001
(60) Divisional application: 04077438.2
(73) Proprietor: International Reagents Corporation, Kobe-shi Hyogo 651-2271 (JP)
(72) Inventor: BABA, Toshiyuki Int. Reagents Corp., Kobe-shi, Hyogo 651-2241 (JP); TABATA, Hiromasa Int. Reagents Corp., Kobe-shi, Hyogo 651-2241 (JP); NAGAMATSU, Katashi Int. Reagents Corp., Kobe-shi, Hyogo 651-2241 (JP); WATAZU, Yoshifumi Int. Reagents Corp., Kobe-shi, Hyogo 651-2241 (JP); AOKI, Ryoji Asahi Chemical Industry Co., Ltd., Tagata-gun, Shizuoka 410-2321 (JP)
(74) Representative: Suèr, Steven Johannes
(86) International application number: PCT/JP1999/002205
(87) International publication number: WO 1999/055850

(56) References cited:
- EP-A- 0 791 658
- GB-A- 866 423
- JP-A- 8 187 095
- JP-A- 51 026 284
- JP-A- 55 141 194
- JP-A- 57 122 795
- JP-A- 60 224 499
- SEGAL H L ET AL: "INTRACTION OF RAT LIVER ENZ ALANINE AMINO TRANSFERASE WITH L PROLINE" BIOCHEMICAL AND BIOPHYSICAL RESEARCH COMMUNICATIONS, vol. 30, no. 1, 1968, pages 63-68, XP001063379 ISSN: 0006-291X
- JACOBI R AND GÖCKERITZ D: "Stabilität und Stabilisierung von Enzymen" PHARMAZIE, VEB VERLAG VOLK UND GESUNDHEIT. BERLIN, DD, vol. 44, no. 10, October 1989 (1989-10), pages 678-685, XP002102673 ISSN: 0031-7144

## Description

### Technical Field

The present invention relates to a method of stabilizing an enzyme contained in a medium and an enzyme composition, with regard to a control substance. The control substance is used to fulfill the following roles, that is, those containing a certain amount of an enzyme as a component are used as the control substance and applied to an analyzer in an test for measuring the amount of the enzyme contained in human blood, whereby the analyzer inspects whether or not an accurate value of the enzyme can be detected. Alternatively, the numerical value of measurement of the control substance is previously obtained, whereby an accurate amount of the enzyme is obtained from the measurement value obtained in an actual specimen by proportioning.

### Background Art

Aspartate aminotoransferase EC 2.6.1.1 (hereinafter abbreviated to AST) is an enzyme capable of producing glutamic acid and oxaloacetic acid from aspartic acid and α - ketoglutaric acid and mainly exists in heart, liver and skeletal muscle, and is a useful index for diagnosis of acute hepatitis, chronic hepatitis and cardiac infarction.

In an ordinary test performed to measure the amount of AST contained in blood, the control substance is often used.

Accordingly, handling of the control substance has become important to secure the stability and reliability of an test value in an ordinary test and to perform a clinical trial to which an accurate and high-level technique is required. For example, in case the measurement value of AST contained in human blood is detected, a control substance containing AST is used. However, it is necessary to sufficiently stabilize the enzyme activity of AST as an unstable enzyme incorporated in the control substance to sufficiently fulfill the roles of the control substance described above.

From such a point of view, the prior arts with the object of stabilizing the enzyme incorporated in the control substance are disclosed in Unexamined Patent Publication (Kokai) No. Sho 55-141194, Unexamined Patent Publication (Kokai) No. Sho 56-148291 and Unexamined Patent Publication (Kokai) No. Sho 57-45453. In these prior arts, ethylene glycol, sucrose or glycerol is used as the stabilizer.

The stabilization of the enzyme using amino acid has hitherto been studied by Harold L. Segal et. al. (Biochemical and Biophysical Research Community cations, Vol. 30 (1), pages 63-68, 1968).

Although there is also a report using amino acid to reduce the turbidity of the control substances, it is considered that amino acid can prevent denaturation of protein in either case.

However, a conventional stabilizer such as ethylene glycol, sucrose or glycerol must be used in high concentration so that the stabilizer exhibits the effect thereof. That is, the concentration of ethylene glycol and that of glycerin must be became higher, for example, about 5 mol/L and 3.3 mol/L, while sucrose must be added in the proportion within a range from 1 to 10%. Therefore, the control substance itself has high specific gravity and/or high viscosity to cause such a problem that the control substance, which should serve as a parameter, has physical properties different from those of human serum. Such a problem leads to the state where an intrinsic object of the control substance itself can not be attained, for example, it produces a factor for causing a difference in measured value between types of a latest automatic analyzer and equipments when applying the control substance to the analyzer because the accuracy of sampling is different from that of common human serum (Japan Clinical Chemistry Society, Scientific Liaison Committee, Clinical Chemistry Vol. 25 (2), pages 135-148, 1996).

### Disclosure of the Invention

To solve the problems described above, the present inventors have studied intensively based on the fact such as roles of amino acid to be fulfilled to the turbidity of the control substances and denaturation of protein. As a result, they have found that valine particularly stabilizes the enzyme AST contained in the control substance, thus completing the method of stabilizing an enzyme and enzyme composition of the present invention.

The present invention provides a method of stabilizing aspartate aminotransferase in at least one medium selected from the group consisting of serum and buffer which comprises adding valine to said medium.

The valine concentration is preferably within a range from 0.5 to 100 mmol/L.

The serum or buffer is preferably a buffer containing a soluble protein.

The soluble protein is preferably at least one soluble protein selected from the group consisting of albumin and gelatin.

Preferably, the soluble protein is albumin in a concentration within a range from 0.5 to 15W/V% , or gelatin in a concentration within a range from 0.5 to 15W/V%.

The present invention also provides an enzyme composition comprising at least one medium selected from the group consisting of serum and buffer, aspartate aminotransferase, and valine.

In the above description, the medium refers to a mother liquor such as solvent or dispersion medium in which the enzyme and stabilizer are dissolved or dispersed, and those prepared by dissolving or dispersing the enzyme and stabilizer in the medium can be used as the control substance. In case the object to be test is contained in human serum, human serum or a similar one prepared by treating the human serum is preferably used as the medium for control substance. That is, it is preferable to select, as the medium, those having properties similar to those of the object to be examined.

As the medium, for example, serum and buffer can be used. The serum refers to human serum, serum of other animals, or treated serums in a broad sense. The serum and buffer may be a buffer containing a soluble protein solution. Hereinafter, aspartate aminotransferase is abbreviated to AST.

Valine as the stabilizer for stabilizing AST is used alone.

In the features described above, those containing AST as the enzyme in the medium can be used as the control substance, typically, in case the amount of AST contained in human blood is measured.

The origin of AST to be incorporated in the control substance includes, but is not specifically limited to, biological substance such as bovine heart, pig heart, human heart, serum, red blood cell and urine, those prepared by cultivating human cells or those prepared by cultivating a transformingcell integrated with a human-derived gene. In this case, the content of AST is controlled within a range from 5 to 1000 U/L, and preferably from 30 to 500 U/L.

In the features described above, when the medium is a buffer, for example, a BES buffer prepared by dissolving bovine serum albumin can be used. However, those prepared by dissolving various substances in buffer can be used to have chemically or physically similar properties corresponding to types and states of the object to be examined (strictly a medium of the object to be examined) to be subjected actually to an examining apparatus. The present invention also includes such a case.

The buffer used in the present invention includes, for example, organic amine and Good's buffers whose pH can be adjusted appropriately within a range from 6.0 to 8.5; and biochemical buffers such as citric acid-sodium diphosphate buffer, hydrochloric acid-sodium veronal-sodium acetate buffer, potassium monophosphate-sodium diphosphate buffer, potassium monophosphate-borate buffer, potassium monophosphate-sodium hydroxide buffer, hydrochloric acid-collidine buffer, hydrochloric acid-sodium veronal buffer, hydrochloric acid-tris(hydroxymethyl)aminomethane buffer, hydrochloric acid-borate buffer, boric acid-sodium carbonate buffer, boric acid-borate buffer, hydrochloric acid-aminomethylpropanediol buffer, ammonium chloride-ammonia buffer, glycine-sodium hydroxide buffer, boric acid-sodium hydroxide buffer, hydrochloric acid-sodium dimethylglycine buffer, borate-sodium hydroxide buffer, borate-sodium carbonate buffer, Sörensen buffer, glycine-sodium chloride-hydrochloric acid buffer, sodium dicitrate-hydrochloric acid buffer, sodium diacetate-sodium hydroxide buffer, borate-sodium hydrochloride buffer, Michaerlis. buffer, sodium veronal-sodium acetate-hydrochloric acid buffer, Clark-Lub's buffer, boric acid-potassium hydrochloride-sodium hydroxide buffer, Atkins-Pantin buffer, Palitzsch buffer, Kolthoff buffer, McIlvaine buffer, Hasting-Sendroy buffer, Britton-Robinson buffer, maleate buffer, tris-maleate buffer, veronal buffer and veronal-acetate buffer. Buffers other than these buffers can also be used as far as they have buffering capacity at the pH within a range from 6.0 to 8.5.

The organic amine buffer includes, for example, diethanolamine buffer, 2-ethylaminoethanol buffer, 2-amino-2-methyl-1-propanol and N-methyl-D-glucamine.

The Good's buffer includes, for example, MES (2-(N-Morphilino)ethanesulfonic acid) buffer, Bis-Tris (Bis(2-hydroxyethyl)iminotris(hydroxymethyl)methane) buffer, ADA (N-(2-Acetamido)iminodiacetic acid) buffer, PIPES (Piperazine-N,N'-bis(2-ethanesulfonic acid) buffer, ACES (N-(2-Acetamido)-2-aminoethanesulfonic acid) buffer, MOPSO (3-(N-Morpholino)-2-hydroxypropanesulfonic acid) buffer, BES (N,N-Bis(2-hydroxyethyl)-2-aminoethanesulfonic acid) buffer, MOPS (3-(N-Morpholino)propanesulfonic acid) buffer, TES (N-Tris(hydroxymethyl)methyl-2-aminoethanesulfonic acid) buffer, HEPES (N-2-hydroxyethylpiperazine-N'-2-ethanesulfonic acid) buffer, DIPSO (3-[N,N-Bis(2-hydroxyethyl)amino]-2-hydroxypropanesulfonic acid) buffer, TAPSO (N-Tris(hydroxymethyl)methyl-2-hydroxy-3-aminopropanesulfonic acid) buffer, POPSO (Piperazine-N,N'-bis(2-hydroxypropanesulfonic acid) buffer, HEPPSO (N-2-hydroxyethylpiprrazine-N-2-hydroxypropane-3-sulfonic acid) buffer, EPPS (N-2-Hydroxyethylpiperazine-N'-3-propanesulfonic acid, another name: HEPPS) buffer, Tricine (Tris(hydroxymethyl)methylglycine) buffer, Bicine (N,N-bis(2-hydroxyethyl)glycine) buffer, TAPS (N-tris(hydroxymethyl)methyl-3-aminopropanesulfonic acid) buffer, CHES (2-(Cyclohexylamino)ethanesulfonic acid) buffer, CAPSO (3-N-Cyclohexylamino-2-ydroxypropanesulfonic acid) buffer and CAPS (3-Cyclohexylaminopropanesulfonic acid) buffer.

When using the organic amine buffer, it may be used as an aqueous medium whose concentration was adjusted within a range from 20 mM to 2 M, preferably from 20 mM to 1 M, and most preferably from 20 mM to 500 mM. Preferable aqueous medium is specifically purified water, and coenzymes, soluble salts, surfactants, stabilizers and antiseptics may also be appropriately incorporated.

When using the Good's buffer or biochemical buffer, it may be used after adjusting the concentration within a range from 20 mM to 1 M, preferably from 20 mM to 500 mM, and most preferably from 20 mM to 300 mM.

In the features described above, when the medium is a soluble protein solution, the soluble protein solution includes aqueous solutions of BSA, human serum albumin (HSA) and gelatin and these aqueous solutions can be used alone or in combination. In this case, the concentration of albumin and that of gelatin are respectively within a range from 0.5 to 15W/V%. When the medium is serum, the buffers described above can also be appropriately used.

Valine is not added in a large amount so that the resulting control substance has neither high specific gravity, nor high viscosity.

The content of valine is preferably within a range from 0.5 to 100 mmol/L. More preferably, the content of valine is controlled within a range from 10 to 20 mmol/L. It is made possible to exert good stabilization effect and to enable physical and chemical properties to bear resemblance to those of serum as the object to be examined by controlling each content within the above range.

It is made possible to exert good stabilization effect and to enable physical and chemical properties to bear resemblance to those of serum as the object to be examined by controlling each content within the above range. Thus, it is made possible to continually make physical and chemical properties of the control substance resemble to those of serum as the object to be examined, secure the stability, obtain the reliability of the resulting data, and eliminates a difference between equipments where the examination is performed.

According to the method of stabilizing AST of the present invention, it is made possible to inhibit denaturation of, and thereby stabilize, AST in a medium by incorporating valine.

It is also made possible to stabilize AST by the stabilizer and to obtain stable and accurate detected data in the examination whose object to be examined is AST . By using valine as the stabilizer to AST , particularly, this enzyme can be sufficiently stabilized by a small content of valine.

According to the method of stabilizing AST of the present invention, it is made possible to use the control substance wherein AST as well as valine as the stabilizer thereof are added in serum as the medium and to detect under good environment which is physically and chemically similar when AST contained in serum is detected by using the medium as serum.

Similarly, AST can be stabilized with valine as the stabilizer in a stable medium such as buffer by using the medium as the buffer. As a matter of course, a control substance having physical and chemical properties similar to those of the object to be examined (solvent) by optionally dissolving various components in the buffer.

In the method of stabilizing AST of the present invention, when using valine, AST can be satisfactorily stabilizing by controlling the valine content within a range from 0.5 to 100 mmol/L.

In the method of stabilizing AST of the present invention, in case the serum or buffer is buffer containing a soluble protein, AST as the stabilizer can be satisfactorily stabilized by valine.

In case the soluble protein is at least one soluble protein selected from the group consisting of albumin and gelatin, AST can be stabilized by valine as the stabilizer in a buffer containing albumin or gelatin. In this case, AST can be preferably stabilized when the concentration of albumin and that of gelatin are respectively within a range from 0.5 to 15W/V%.

According to the enzyme composition of the present invention, there can be provided an enzyme composition, which is not liable to cause denaturation of AST and can exist in the stable state, by incorporating AST, and valine in at least one medium selected from the group consisting of serum and buffer. Therefore, it is made possible to obtain stable and accurate detected data by using such an enzyme composition, as the control substance, in the examination whose object to be examined is AST.

### Best Mode for Carrying Out the Invention

Examples of the preparation of a control substance comprising valine as a stabilizer and AST as an enzyme component according to the present invention will now be described.

Using human serum (manufactured by TORINA CO. (Switzerland)), an endogenous enzyme is devitalized by previously subjecting to a heat treatment at 56°C for four hours and then sterilized by filtration using a membrane filter of 0.2 µm in pore size (solution thus obtained is referred to as a human serum base). For example, 10 mmol/L of valine is dissolved in this human serum base (medium) and a certain amount of AST is dissolved furthermore, thereby making it possible to obtain a control substance containing valine as a stabilizer and AST.

Examples of the preparation of a control substance comprising valine as a stabilizer, AST as an enzyme component and a buffer as a medium according to the present invention will now be described.

Bovine serum albumin of 3W/V% (manufactured by INTERGEN CO. (U.S.A)) is incorporated in a BES buffer (20 mmol/L) and then sterilized by filtration (pH 7.4, solution thus obtained is referred to as a BSA base). For example, 10 mmol/L of valine is dissolved in this BSA base and a certain amount of AST is dissolved furthermore, thereby making it possible to obtain a control substance containing valine as a stabilizer and AST in the buffer medium.

Furthermore, the control substance obtained by the method of the present invention is in the state of liquid on common use, but may be in the state other than liquid, such as freeze-dried product, cold-stored product and frozen liquid product.

### Examples

The following examples further illustrate the present invention, but the present invention is not limited by the examples.

### Example 1

Confirmation of stabilization effect of various amino acids on AST in control substance

Using a human serum base and a BSA base as a medium, control substances were prepared respectively by adding, as a stabilizer, no amino acid, valine (10 mmol/L) as amino acid, proline (10 mmol/L) or other amino acid (10 mmol/L, provided that 1 mmol/L in case of tyrosine) to the respective mediums and then adding, as an enzyme, AST (about 100 U/L) .

The respective control substances thus obtained were stored at 45°C for four days and the residual activity of AST contained in the respective control substances was measured.

AST used herein is AST derived from a human liver gene recombinant (hereinafter referred to as r-AST) manufactured by ASAHI CHEMICAL INDUSTRIES CO., LTD. (manufacturing No. T-70).

Furthermore, the human serum base used herein is a human serum base prepared by previously subjecting human serum to a heat treatment at 56°C for four hours thereby to devitalize an endogenous enzyme, followed by sterilization by filtration using a membrane filter of 0.2 µm in a pore diameter. The BSA base used herein is BES buffer (20 mmol/L) containing 3W/V% BSA.

With regard to the residual activity of AST, the residual activity of AST was measured by using an AST reagent L "KOKUSAI" manufactured by INTERNATIONAL REAGENTS CORPORATION.

The results are shown in Table 1.

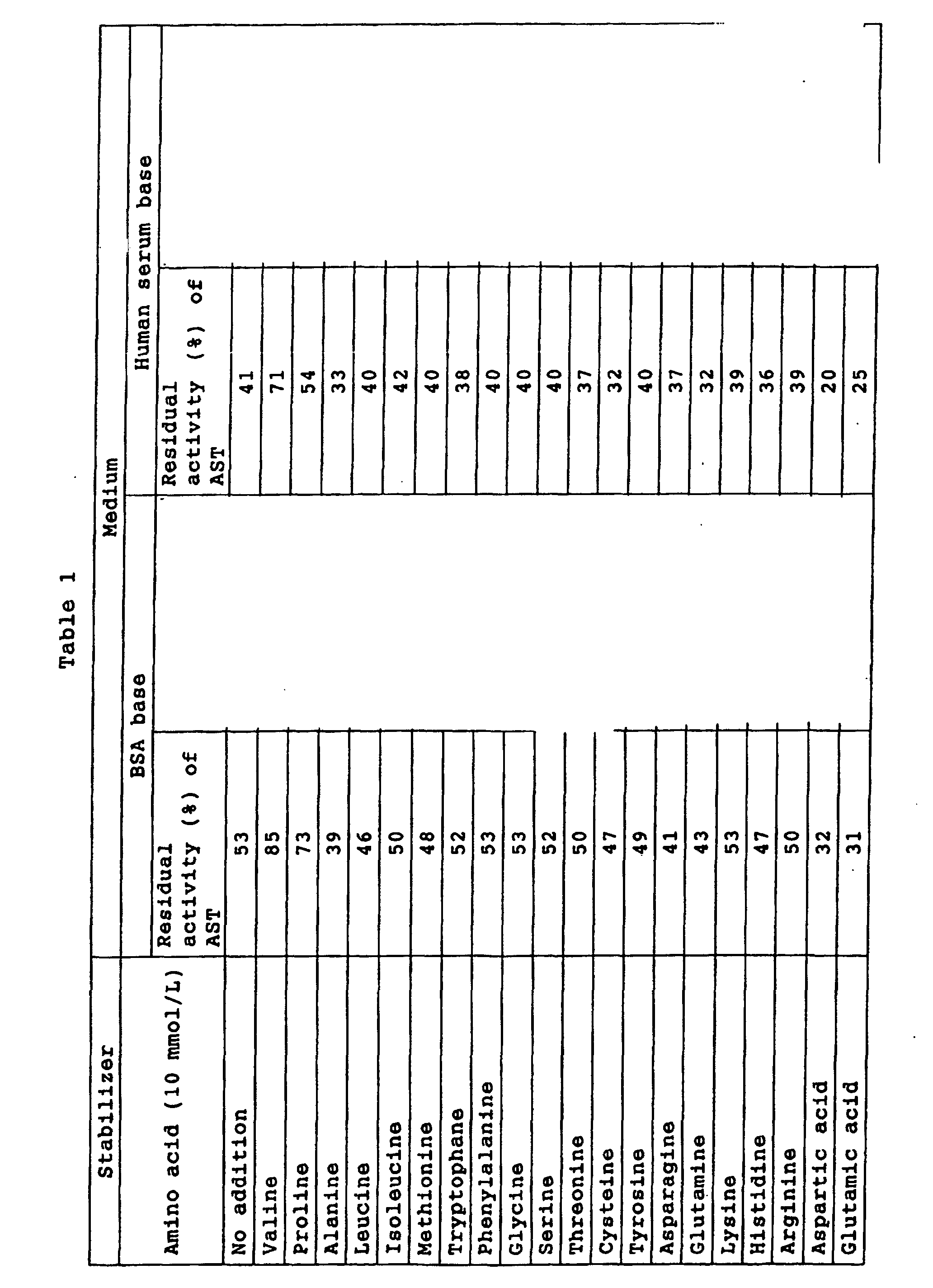

As is apparent from Table 1, valine exerted preferable stabilization effect on AST .

### Example 2

Confirmation of stabilization effect of valine on AST in control substance

valine and r-AST were added to a human serum base and a BSA base in the concentration of about 100 U/L and, after storage at 45°C for four days, the residual activity was measured. The measurement of the activity of AST was performed in the same manner as in Example 1.

The results are shown in Table 2.

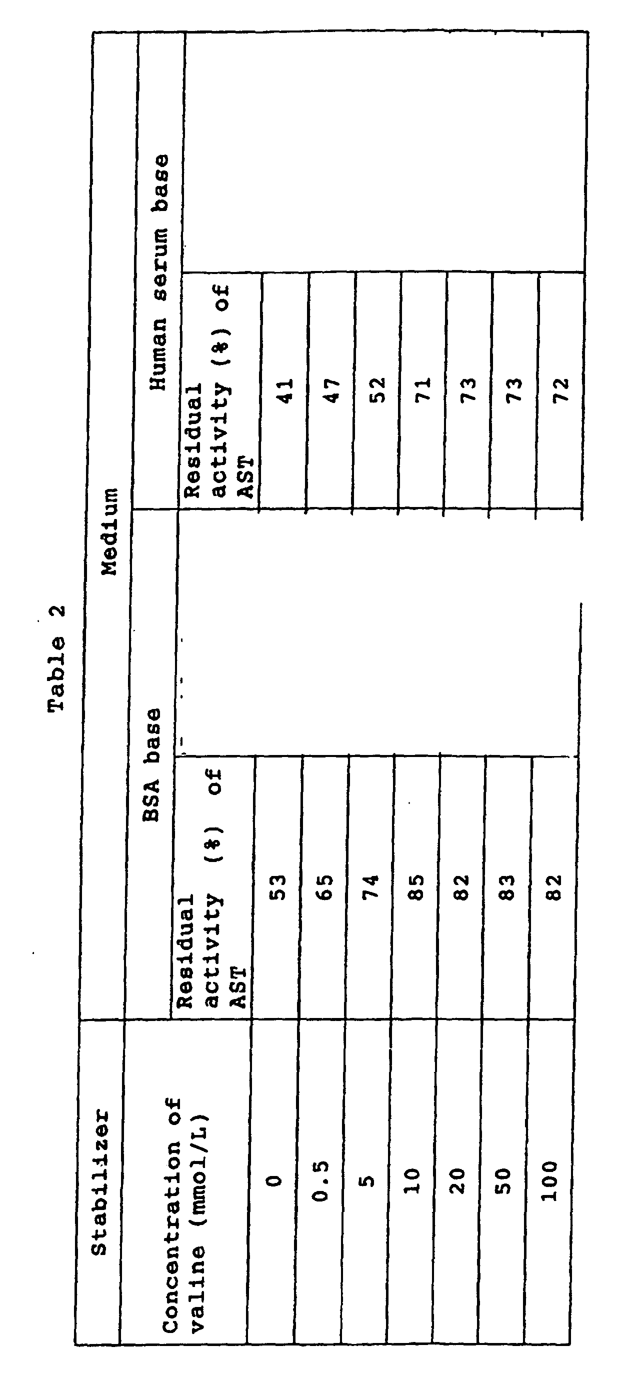

As is apparent from Table 2, the residual activity of AST was 53% in case of no addition of valine in the BSA base, while the residual activity of AST became 65% by adding 0.5 mmol/L of valine, thus improving the stability. The residual activity of AST was 85% when the concentration of valine is 10 mmol/L, while the residual activity of AST was 82% when the concentration of valine is 20 mmol/L. Furthermore, the residual activity of AST was 82% when the concentration of valine is 100 mmol/L.

Similarly, the residual activity of AST was 41% in case of no addition of valine in the human serum base, while the residual activity of AST was 47% by adding 0.5 mmol/L of valine, thus improving the stability. The residual activity of AST was 71% when the concentration of valine is 10 mmol/L, while the residual activity of AST was 73% when the concentration of valine is 20 mmol/L.

Furthermore, the residual activity of AST was 72% when the concentration of valine is 100 mmol/L.

As is apparent from the above description, the concentration of valine is preferably controlled within a range from 0.5 to 100 mmol/L, and more preferably from 10 to 20 mmol/L.

### Industrial Applicability

A method of stabilizing an enzyme and an enzyme composition of the present invention are concerned with a control substance used in medical examination and are capable of sufficiently stabilizing AST contained in serum, a buffer or a medium such as soluble protein solution. Thus, the present invention has industrial applicability by providing a method of and a material for obtaining accurate and stable examined data in the field of the medical clinical examination.

## Claims

1. A method of stabilizing aspartate aminotransferase in at least one medium selected from the group consisting of serum and buffer, the method comprising adding valine to said medium.

2. A method according to claim 1, wherein the valine concentration is within a range from 0.5 to 100 mmol/L.

3. A method according to claim 1 or 2, wherein said serum or buffer is a buffer containing a soluble protein.

4. A method according to claim 3, wherein said soluble protein is at least one soluble protein selected from the group consisting of albumin and gelatin.

5. A method according to claim 4, wherein the concentration of said albumin is within a range from 0.5 to 15W/V%, or wherein the concentration of said gelatin is within a range from 0.5 to 15W/V%.

6. An enzyme composition comprising at least one medium selected from the group consisting of serum and buffer, aspartate aminotransferase, and valine.

7. An enzyme composition according to claim 6 wherein the valine concentration is within a range from 0.5 to 100 mmol/L.

8. An enzyme composition according to claim 6 or 7 wherein said serum or buffer is a buffer containing a soluble protein.

9. An enzyme composition according to claim 8 wherein said soluble protein is at least one soluble protein selected from the group consisting of albumin and gelatin.

10. An enzyme composition according to claim 9 wherein the concentration of said albumin is within a range from 0.5 to 15W/V%, or wherein the concentration of said gelatin is within a range from 0.5 to 15W/V%.

## Patentansprüche

1. Verfahren zum Stabilisieren von Aspartat-Aminotransferase in mindestens einem Medium, das aus der Gruppe ausgewählt ist, die aus einem Serum und einem Puffer besteht, wobei das Verfahren die Zugabe von Valin zum Medium umfasst.

2. Verfahren nach Anspruch 1, worin die Valinkonzentration im Bereich von 0,5 bis 100 mmol/L liegt.

3. Verfahren nach den Ansprüchen 1 oder 2, worin das Serum oder der Puffer ein Puffer ist, der ein lösliches Protein enthält.

4. Verfahren nach Anspruch 3, worin das lösliche Protein mindestens ein lösliches Protein ist, das aus der Gruppe ausgewählt ist, die aus Albumin und Gelatine besteht.

5. Verfahren nach Anspruch 4, worin die Konzentraten des Albumins im Bereich von 0,5 bis 15 % (Gew./Vol.)liegt, oder worin die Konzentration der Gelatine im Bereich von 0,5 bis 15 % (Gew./Vol.) liegt.

6. Enzymzusammensetzung, umfassend mindestens ein Medium, das ausgewählt ist aus der Gruppe, die aus Serum und Puffer, Aspartat-Aminotransferase und Valin besteht.

7. Enzymzusammensetzung nach Anspruch 6, worin die Valinkonzentration im Bereich von 0,5 bis 100 mmol/L liegt.

8. Enzymzusammensetzung nach den Ansprüchen 6 oder 7, worin das Serum oder der Puffer ein Puffer ist, der ein lösliches Protein enthält.

9. Enzymzusammensetzung nach Anspruch 8, worin das lösliche Protein mindestens ein lösliches Protein ist, das aus der Gruppe ausgewählt ist, die aus Albumin und Gelatine besteht.

10. Enzymzusammensetzung nach Anspruch 9, worin die Konzentration des Albumins im Bereich von 0,5 bis 15 % (Gew./Vol.) liegt, oder worin die Konzentration der Gelatine im Bereich von 0,5 bis 15 % (Gew./Vol.) liegt.

## Revendications

1. Procédé de stabilisation de l'aspartate aminotransférase dans au moins un milieu choisi dans le groupe consistant en sérum et tampon, le procédé comprenant l'addition de valine audit milieu.

2. Procédé selon la revendication 1, dans lequel la concentration en valine se situe dans une plage de 0,5 à 100 mmoles/l.

3. Procédé selon l'une des revendications 1 ou 2, dans lequel ledit sérum ou tampon est un tampon contenant une protéine soluble.

4. Procédé selon la revendication 3, dans lequel ladite protéine soluble est au moins une protéine soluble choisie dans le groupe consistant en albumine et gélatine.

5. Procédé selon la revendication 4, dans lequel la concentration de ladite albumine se situe dans une plage de 0,5 à 15 % p/v, ou dans lequel la concentration de ladite gélatine se situe dans une plage de 0,5 à 15 % p/v.

6. Composition enzymatique comprenant au moins un milieu choisi dans le groupe consistant en sérum et tampon, aspartate aminotransférase et valine.

7. Composition enzymatique selon la revendication 6, dans laquelle la concentration en valine se situe dans une plage de 0,5 à 100 mmoles/l.

8. Composition enzymatique selon l'une des revendications 6 ou 7, dans laquelle ledit sérum ou tampon est un tampon contenant une protéine soluble.

9. Composition enzymatique selon la revendication 8, dans laquelle ladite protéine soluble est au moins une protéine soluble choisie dans le groupe consistant en albumine et gélatine.

10. Composition enzymatique selon la revendication 9, dans laquelle la concentration de ladite albumine se situe dans une plage de 0,5 à 15 % p/v, ou dans laquelle la concentration de ladite gélatine se situe dans une plage de 0,5 à 15 % p/v .
